# EUROPEAN PATENT SPECIFICATION

(11) **EP 4 221 595 B1**
(45) Date of publication and mention of the grant of the patent: **17.07.2024**
(21) Application number: 21782597.5
(22) Date of filing: 09.09.2021
(51) Int. Cl.: A61B 5/389

(54) **INTEGRATED SYSTEM FOR DETECTION AND PROCESSING ELECTROMYOGRAPHIC SIGNALS**
INTEGRIERTES SYSTEM ZUR DETEKTION UND VERARBEITUNG ELEKTROMYOGRAPHISCHER SIGNALE
SYSTÈME INTÉGRÉ DE DÉTECTION ET DE TRAITEMENT DE SIGNAUX ÉLECTROMYOGRAPHIQUES

(30) Priority: 29.09.2020 IT 202000022933
(43) Date of publication of application: 09.08.2023
(73) Proprietor: BIONIT LABS SRL, 73100 Lecce (IT)
(72) Inventor: MACRÌ, Chiara, 73057 Taviano (LE) (IT); GAETANI, Federico, 40026 Imola (BO) (IT); COLAZZO, Giorgio, 73049 Ruffano (LE) (IT)
(74) Representative: Conversano, Gabriele
(86) International application number: PCT/IB2021/058209
(87) International publication number: WO 2022/069976

(56) References cited:
- EP-A2- 1 779 820
- WO-A1-2012/141714
- WO-A1-2012/161657
- WO-A1-2016/207471
- CN-A- 106 491 128
- CN-A- 108 670 244
- CN-B- 110 141 239

## Description

The present invention relates to an integrated system for detecting electromyographic signals, processing such signals and controlling devices such as prostheses, orthoses or exoskeletons.
- The word prosthesis indicates an artificial device apt to substitute a missing part of the body (a limb or a joint), or to integrate a damaged one;
- the word orthosis indicates a corrective device for the limbs of the body, which can be applied to them, but which does not substitute them;
- the word exoskeleton indicates an outer device able to increase the physical capacity of the user wearing it.

The mechanical portion (including suitable moving means) of anyone of these kinds of devices is referred to as "actuator". So, in the present invention the word actuator indicates a wearable device able to carry out a movement in substitution of the movement carried out by the user, in addition to such movement or for correcting it.

### State of the art

At the state of the art, there are known many embodiments of prostheses provided with moving means controlled by the analysis of electromyographic signals. Some examples are described in documents EP1857081, EP2525746, EP2688523, EP3150120B1.

In these ones and in other documents known at the state of the art, electromyography sensors are described, which are integrated in various manners inside the prostheses or "socks" worn by the user. At the state of the art, there are also known systems made up of a variable number of surface electromyography electrodes, positioned in contact to the user skin, that detect the electromyographic signals generated by the muscle contraction and send such signals to the electronic device carrying out the function of prosthesis control.

An example is reported in document US2019343661, where it is described a system for the configuration of a myoelectric-controlled prosthetic device made up of a storage and a variable number of surface electrodes made up of metal contacts in contact to the user skin, positioned along the stump circumference and arranged for detecting the electromyographic signal. Such system has many drawbacks, the first one of which being linked to the fact that being the electrodes remote/passive the AD conversion does not occur at the metal contacts and so at the muscle tissue of the subject, but in a controller positioned downstream of the system. So, the distance covered by the electromyographic signal is such that the probability of coupling with other outer analog signals increases and that SNR increases. Another limit of such system is represented by the fact that the electrodes are necessarily positioned around the stump at the same distance to each other, according to a predefined position which does not allow the customization and the individuation of the most suitable muscle sites so that the intramuscular crosstalk is reduced and the spatial configuration of the system is optimized with respect to the user anatomy.

The last limit relates also to another system known at the state of the art, described in document US9299248, in which an armband is described which is positioned on the forearm and provided with a variable number of surface electrodes for detecting the electromyographic signal of the user. Since it is an armband, the electrodes are necessarily arranged radially at predefined distance to each other and so it is not possible to optimize their positioning. Moreover, the system described in such document comprises a module apt to provide haptic feedback to the user; such module can be represented by a little motor generating a vibration. Anyway, the feedback provided consists exclusively in the confirmation of the recognition of gestures carried out by the user and does not give other information allowing to facilitate the device usage.

Document US10152082B2 describes a wearable electronic device, with variable circumference, that is provided with a series of housings to house many kinds of sensors, among which electromyography sensors, in number between two and eight, with necessarily radial arrangement of the electrodes and with predefined distance to each other.

Anyway, at the best of the present inventors' knowledge, no one of the devices known at the state of the art allows to solve a series of technical problems at the same time:
- the full possibility of configuration of number and arrangement of the used electromyography sensors;
- the reduction of disturbances in the acquisition of the electromyographic signal;
In fact, all the kinds of electromyography sensors integrated in the sock of the device or in the armband are in fixed position, and their arrangement is not customizable for the single user. Moreover, the physical distance between their position and the digitalizing device makes it impossible to limit disturbances in the transmission of the analog signal.

On the other hand, sensors not integrated in the sock (which are generally enclosed in independent and repositionable envelopes) known at the state of the art have the same defects in terms of disturbances and interferences in the transmission of the analog signals. Other relevant prior art is disclosed in CN 110141239 B, WO 2012/161657 A1, CN 106491128 A, CN 108670244 A, WO 2012/141714 A1, EP 1779820 A2, WO 2016/207471 A1.

### Aim of the invention

Aim of the present invention is to provide an integrated system for detecting and processing electromyographic signals and for controlling an actuator, which overcomes the limits linked to embodiments known at the state of the art. More in particular, the present invention provides an integrated system for detecting and processing electromyographic signals and for controlling an actuator comprising at least an electrode which is easily repositionable, so that it is adapted to the anatomy of any user, which is configured to acquire electromyographic signals and to carry out their digital conversion while reducing disturbances and interference at minimum.

The disclosed integrated system allows also for detecting and processing electromyographic signals, and for controlling an actuator which is easily reconfigurable with regard to number and position of the used electrodes.

### Brief description of the invention

The invention is disclosed in independent claim 1. The present disclosure provides an integrated system for detecting and processing electromyographic signals and for controlling an actuator (40) comprising at least a mechanical moving means and configured to carry out at least a kind of movements as a function of at least an input signal, said system comprising:
- a controller or control module (10),
- at least an electrode module (20) configured to acquire, amplify, convert and process electromyographic signals (20),
- a supplying module (30), configured to provide an electric supply with predetermined voltage and current to said controller (10) and to said at least one electrode module (20), said controller (10), said at least one electrode module (20) and said supplying module (30) being connected in parallel on the same bus, characterized in that said electrode module (20) comprises:

- at least a sensor (21) configured to detect a signal on the skin deriving from the muscle contraction,
- amplification means (22) of the signal detected by said at least one sensor (21),
- analog/digital conversion means, configured to convert the analog signal in digital signal, amplified by said amplification means (22),
- electronic means (24) on which computer programs or electrode firmware are loaded, configured to process the signal acquired by said at least one sensor (21).

### Description of the figures

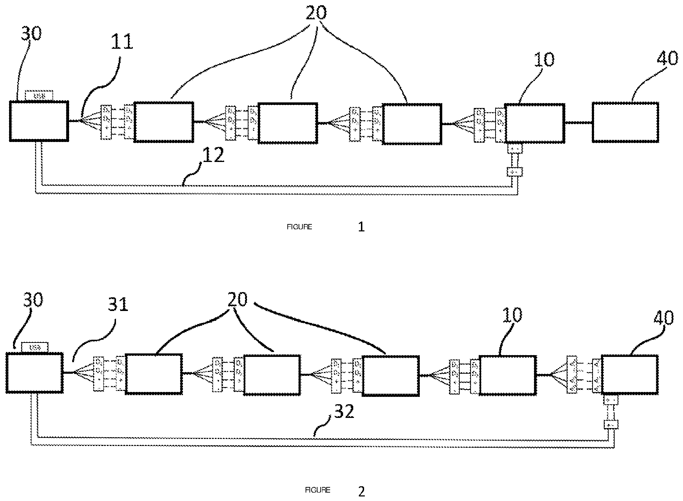

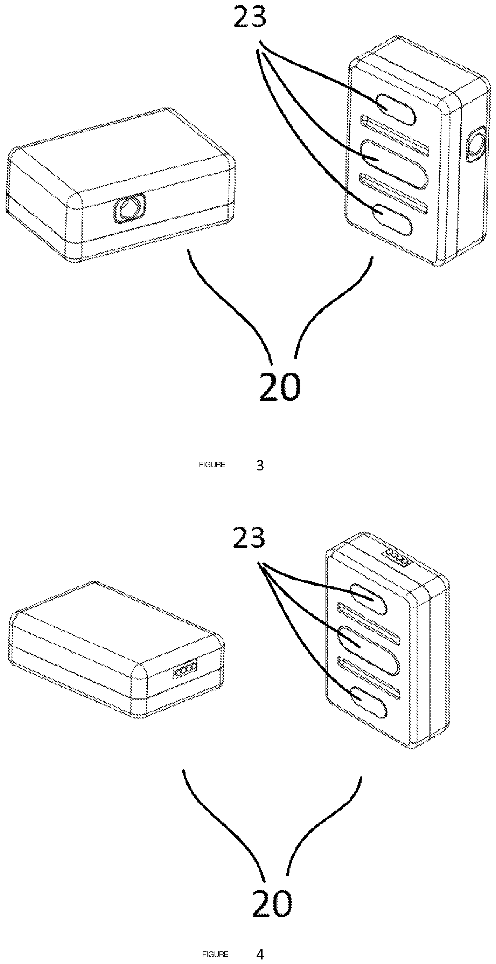

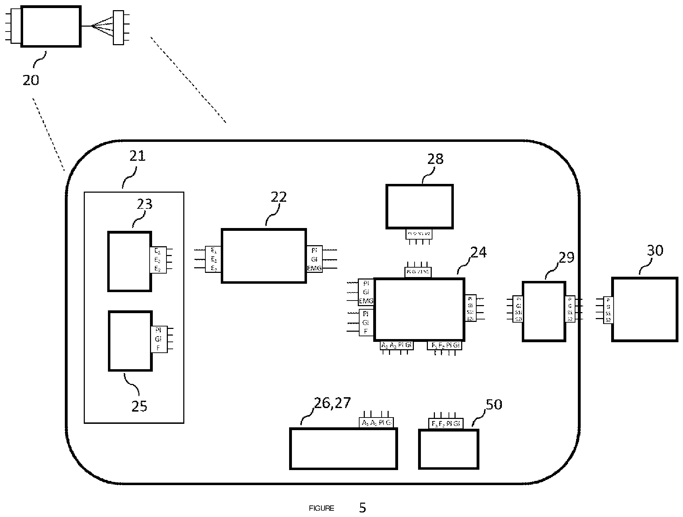

Figures 1 and 2 show two schematic views of two preferred embodiments of the device, wherein the control module (10) supplies the actuator (40) directly, and wherein, vice versa, the actuator (40) is supplied by the supplying module (30); figures 3 and 4 show views of two embodiments of the envelope of the electrode module. Figure 5 shows schematically the inner architecture of the electrode module.

### Detailed description of the device

As it is shown in the appended figures, the system comprises:
- a controller or control module (10),
- at least a device for acquiring, amplifying, digitally converting and processing electromyographic signals (20), in the following defined as electrode or electrode module for brevity,
- a supplying module (30), configured to provide an electric supply with predetermined voltage and current to said controller (10) and to said electrodes.

Preferably, the system comprises a plurality of electrodes (20).

It is to be specified that in the present disclosure, for "module" it is intended a device connectable to other devices by means of digital connections with bus logic, configured to carry out some specific functions and to share the electric supply with the other modules.

As yet said, the modules usable in the system according to the present disclosure are of three kinds:
supplying module (30), electrode module (20), control module (10).

Moreover, in the present disclosure for "actuator" (40) it is intended a prosthetic device, an orthosis or exoskeleton, configured to be able to carry out at least a kind of movements as a function of at least an input signal.

As it is known, a prosthesis can also comprise more than one moving means, and can be configured to carry out a plurality of different movements as a function of one or more input signals. For example, in case of hand-wrist prosthesis, and as a function of how the mechanism of the prosthesis is realized, movements of finger flexion and extension, wrist flexion and extension and pronosupination of the same are possible.

The controller (10), the electrodes (20) and the supplying module (30) are connected to each other in parallel on the same bus.

The connection of each module to the modules connected thereto occurs by means of at least 3 terminals, at least one of which being used for the transmission of the digital signal and two of which being used for the transmission of the positive pole and negative pole of the electric supply.

Moreover, the supplying module (30) comprises preferably, in addition to the connection (31) supplying all the modules connected as a chain and communicating with them, also another electric connection (32) configured to supply said actuator (40) directly or by means of said control module (10) .

To such aim, preferably but not limitingly, said control module (10) comprises, in addition to the connection (11) by means of which it communicates with and shares an electric supply with the remaining modules, also another electric connection (12) configured to be supplied directly from said supplying module.

The separation of the modules supplying - which is functional exclusively for the management of the functioning of the electronics thereof - from the supplying of the actuator - which instead requires to transport the power needed for the actuation of the various movements - allows to reduce the noise associated to less filtered power supplies, to increase the robustness of the system while keeping the control functions supplied by the low power section in case of first failure of the higher power portion, to reduce through the physical separation of wires disturbances induced in the lower power wires.

After showing the connection modes of the various modules inside the system, it is now possible to show the functioning of each module.

The electrode module (20) according to the disclosure comprises at least a sensor (21) configured to detect a signal on the skin surface deriving from the muscle contraction. In a first embodiment said sensor is an electromyography sensor (23), comprising at least two electrodes configured to detect a difference of potential between two distinct skin regions; in a second embodiment said sensor is a force myography sensor (25) comprising a capacitive pressure sensor configured to measure the pressure exerted on the electrode by the muscle under the skin region where the electrode module is installed.

In a third embodiment, the module comprises both the just described sensors.

Preferably, the electrode module (20) comprises further a magnetic insulating module of the supply (29) which has the function to decouple electrically the user from the supplying module (30) and to reduce the noise associated to the supply and a monitoring system (28) of the supply apt to decouple the board from the skin in case of overcurrents and/or over/undervoltages.

The electrode module (20) comprises also amplification means (2) of the signal detected by said at least one sensor (21), and analog/digital conversion means, configured to convert the analog signal in digital signal, amplified by said amplification means (22).

The electrode module (20) is characterized in that the position of the analog/digital conversion means is at the acquisition position, between the electrodes (or the pressure sensor) acquiring the signal and the analog/digital converter filtration steps or other processing steps not being interposed, except for an amplification step of the signal (optional step in case of pressure sensor). In this way, electromagnetic interferences in the detected signal are reduced.

In case the sensor provided is an EMG sensor (23), such sensor has at least two conductive, preferably metallic surfaces, positioned on the outer side of the envelope of the electrode module (20), in contact with the skin.

Such conductive surfaces are preferably three in number, as it is shown in the appended figures 3 and 4.

In this case, the A/D conversion means are installed on a printed circuit board, contained inside the same envelope of the electrode module (20) and in electric contact with the inner portion of said conductive surfaces.

So, the conductive portion of the EMG sensor has a portion facing outwards of the envelope of the electrode module and a second portion facing inwards.

In case the sensor provided is a capacitive force sensor (25), the reading circuits of the capacity are installed on the same printed circuit board of the analog/digital conversion means, such board being enclosed inside the same envelope of the electrode module (20).

The electrode module (20) comprises further electronic means (24) on which computer programs are loaded (in the following also indicated as electrode firmware), configured to process the signal acquired by said at least one sensor (21) configured to detect a signal on the skin surface deriving from muscle contraction.

Preferably, but not limitingly, said electrode module comprises also temperature sensors (26) and/or humidity sensors (27), configured to detect temperature and humidity relating to the skin region where the electrode is positioned. In this case, said electronic means (24) are also configured to acquire and process the signals detected by said temperature sensors (26) and/or humidity sensors (27).

Preferably, but not limitingly, the previously described system comprises also at least a module for generating a vibro-tactile feedback (50), connected in parallel with said digital communication bus.

In a first embodiment, said module for generating a vibro-tactile feedback comprises a generator of mechanical vibrations, in a second embodiment said module for generating a vibro-tactile feedback (50) comprises a device for transcutaneous electric stimulation.

The electrode firmware is configured to carry out the following operations:
(251) acquiring the signals deriving from said at least one sensor (21) configured to detect a signal on the skin surface deriving from the muscle contraction and converted in digital ones by said analog/digital conversion means,
(252) carrying out the frequency transform (FFT), Wavelet Transform, Power Spectrum computing of said signals, thus obtaining at least a frequency spectrum relative to said at least one signal,
(253) carrying out notch operations on the acquired signal by eliminating from the signal the frequencies relative to possible interferences deriving from the electric supply.
   It is to be specified that the notch filtering execution on the digital signal allows to vary at one's pleasure the notch frequency to be used, by simple software configuration,
(254) carrying out frequency filtering operations on said frequency signal, by applying predetermined amplitude band-pass filters.
(255) communicating with the other modules provided in the device, and in particular with said control module (10), in order to transmit the signal obtained through the just described processing, as well as preferably the signals acquired by said pressure and/or temperature signals.

Moreover, the electrode firmware is also preferably configured to modify the gain and offset of the analog/digital conversion step as a function of the control set by the user.

This allows to obtain a correct conversion of the signal in case of using by users with a compromised muscle tone, thus enlarging the range of users who can use the control device.

The electrode firmware is also configured to receive in input by said control module (10) and by means of said communication means, information about the gain and offset to be used in the analog/digital conversion step, the notch frequency to be used in the signal processing and the passband of the frequency filter to be applied.

The electrode firmware is also configured to receive in input by said control module (10) and by means of said communication means, information about the sampling frequency of the signal by said at least one sensor (21) and the sending frequency of the signal processed to said control module (10) .

Moreover, said electrode firmware is preferably configured to compare the shape of the frequency spectrum of the signal detected by said at least one sensor (21) configured to detect a signal on the skin surface with the shape of at least a reference spectrum, to individuate the presence of anomalous peaks of the signal, indicating the presence of interferences (electromagnetic coupling with outer sources). In such case, the firmware sends a warning signal to said control module, so that the user can evaluate if to modify the cutting frequencies of said bandpass filter.

Said firmware is also configured to detect the presence of peaks under 10 Hz of frequency indicating the presence of electrode-skin movements. In such case the firmware sends a warning signal to said control module so that the user can check the correct positioning and clamping of the device.

Moreover, said firmware is configured also to detect periodically a central frequency of the PSD spectrum of said signal, and to send a warning signal to said control module in case said frequency shifts over the time at ever lower frequencies. Such shifting indicates muscle fatigue of the user.

As yet said, the device comprises preferably a plurality of electrode modules (20), which can be arranged in different positions to each other, with no mutual positioning constraints.

In particular, in order to be connected to the system each electrode module needs only an at least 3 contacts wiring to the module preceding it and to the module following it (regardless of the kind of module connected), so that both electric supplying contacts and digital communication contacts can be connected.

Wiring, having to transport unimportant intensity electric currents, being functional only for supplying little electronic devices, can be obtained with wires with extremely little section, with lengths needed to position the electrodes in the sites more significant for the anatomy of the subject wearing the device.

It is to be specified that this possibility of customization is possible only by means of the features of the just described electrode module, which integrates the functions of signal acquisition, amplification, conversion and filtration.

Moreover, preferably, the lines of the digital bus and low-power supply are insulated by magnetic coupling, so that the user is isolated from electrostatic discharges, leakage currents or direct coupling with the electric network in case of battery recharge while the user wears the device, since the electrodes are in direct contact with the skin of the user. This allows to obtain an insulation which guarantees more safety when the user connects the supplying module to an outer supply source (for example: coupling to the electric network by means of the battery charger).

All the just described components for the electrode module (20) are preferably enclosed inside an envelope with waterproof and powder resistance features and apt to guarantee its functioning without damage even in presence of natural humidity, splashes or sweating. Said envelope has preferably waterproof and power resistance features at least equal to IP67 standard, and to such aim any type of electric connection among the known ones at the state of the art can be used for wiring. The observance of the waterproof and powder resistance features of the envelope is applied also to other kinds of modules usable with the device.

The supplying module (30) comprises at least a rechargeable electric battery, provided with a suitable connector for the recharge by means of connection to the electric network, preferably by means of a common outer voltage transformer with respect to the supplying module.

The controller (10) comprises control electronic means on which computer programs are loaded (in the following also indicated as controller firmware), digital communication means (11) configured to communicate with other electrodes, and at least an output (13) for the control of an actuator.

Said at least one output for the control of an actuator can comprise an output of analog kind, or an output of digital kind.

The control module (10) comprises also outwards communication means, configured to communicate with outer processing means where respective computer programs are loaded configured to allow the user to communicate with said control means by means of a suitable user interface.

As yet said, the control module (10) can be configured in two modes.

In a first embodiment the control module (10) controls directly at least an actuator (40), providing the same with both the control signal and the power needed for supplying motors included in the actuator.

To such aim, the control module (10) comprises hardware means for electronic control of at least a power actuator and respective computer programs (firmware) for closed/open loop control of at least an actuator (40).

In a second embodiment, the control module (10), unlike the first configuration, does not control the actuator/actuators directly, but it does it by means of an outer controller, configured to implement the power control of at least an actuator (40) .

Such outer controller is configured to receive in input different kinds of analog and/or digital signals, and to provide the actuator with control signals and the power needed for moving the motors included in the actuator.

In this embodiment the control module (10) acts as real interface between the system according to the disclosure and actuators of third preexisting parts, thus guaranteeing a functioning compatibility with them. The control module (10) is also configured to carry out the supervision function of the system, by monitoring continuously the presence of error messages, warning coming from all the nodes connected to the bus together with the control of the interactions with the outer environment.

It is also able to individuate automatically the modules connected to the chain when these are connected, and to manage their mutual interaction as a consequence.

In particular, the bus allows one or more electrode modules (20) to be hot connected, while the system is functioning. This is possible thanks to initialization packets which each module is able to exchange with the controller at the moment it is connected to the system.

After a predetermined configuration time, the module will acquire an address, which will remain fixed, also after the system restart. The address will be useful to communicate with the controller and with all the modules yet present on the bus. At the same way, a module can be hot removed from the system.

Through acknowledgment techniques, implemented at low level, the controller is able to detect the presence of a module and to ask the re-transmission of the information, if corrupted. This occurs continuously during the normal functioning of the system and it is useful to adapt the system behaviuor according to the modules provided.

Another advantage of the system according to the invention is that the system is self-calibrating, since each electronic module (20), thanks to a calibration procedure, is able to detect the signals associated to the basic state (i.e. the rest state) and to use them to eliminate the portion of signal not giving any useful information for movement recognition.

In particular, the self-calibration procedure is made up of two steps.

In a first step the controller records the basal signals acquired by the electrode modules (20) during a muscle rest condition of the user and adapts the acquisition parameters (gain, offset, etc.) of the A/D converter provided on each electrode module (20) and the digital filtering parameters, so that the SNR is improved for each electrode module (20).

During such step the controller is configured to signal the user to remain at rest. This occurs by means of a signaling device (buzzer, display, LED, haptic feedback etc.) or by means of a digital communication protocol with an outer HMI (human machine interface).

In the second step, the controller controls the ranges of the acquisition parameters of the A/D converter and digital filtering, so that the calibration is adapted automatically as a function of the observed time window.

To such aim, the control module is configured to detect periodically the range of acquisition parameters of the A/D converter and digital filtering, so that the calibration is adapted automatically as a function of the observed time window. In particular, a procedure of self-calibration is carried out, subdivided in two steps:
a) at each first starting, or recalibration of the system the user is asked to remain in rest condition (rest) for a time interval of predetermined duration Δ*T*.

It is to be specified that rest condition means a condition in which the subject is asked to remain still, with relaxed muscles, in an inactivity state, thus avoiding contracting muscles, avoiding exerting pressure on the portions of the prosthesis and avoiding activating possible sensors provided.

In such condition, the controller on board of the electrode keeps the preset gain and notch values while recalculating, to optimize SNR of the signal, the offset parameters, passband and resolution of the A/D quantization. For each of said three parameters, starting from a predetermined maximum range a dichotomic research of the optimal values is carried out evaluating SNR after each variation of each parameter, up to maximum 5 iterations;
b) after this first step, the user can freely use the prosthesis.

During the functioning, the electrode module analyzes the EMG and/or FMG signals according to preset time windows.

Each window is analyzed and clustered by the control module which communicate the intention of the user.

In case the precision, the recall and F1 score do not satisfy the safety requirements in the prediction of the user intention as a function of the stored calibration, the control module communicates this event through a warning and the electrode module/modules carries/carry out a rapid research of the optimal offset parameters, passband and resolution of the A/D quantization to reach the safety thresholds needed for the actuator moving.

## Claims

1. Integrated system for detecting and processing electromyographic signals and for controlling an actuator (40), comprising at least a mechanical moving means and configured to carry out at least a kind of movements as a function of at least an input signal,
said system comprising:
- a control module (10),
- at least an electrode module (20) configured to acquire, amplify, convert and process electromyographic signals (20),
- a supplying module (30), configured to provide an electric supply with predetermined voltage and current to said control module (10) and to said at least one electrode module (20),
said control module (10), said at least one electrode module (20) and said supplying module (30) being connected in parallel on the same bus,
wherein :
said electrode module (20) comprises:
- at least one sensor (21) configured to detect a signal on the skin surface deriving from muscle contraction, said sensor (21) being an electromyography sensor (23) having at least two conductive surfaces positioned on the outer side of the envelope of said electrode module (20), so that they are configured to be in contact with the skin,
- amplification means (22) of the signal detected by said at least one sensor (21),
- analog/digital conversion means, installed on a printed circuit board contained inside the envelope of said electrode module (20) and in electric contact with the inner portion of said conductive surfaces configured to convert the analog signal in digital signal, amplified by said amplification means (22),
- electronic means (24) on which electrode firmware is loaded, configured to process the signal acquired by said at least one sensor (21),
said electrode firmware being configured to receive in input by said control module (10) information about the gain and offset to be used in the analog/digital conversion step, the notch frequency to be used in the signal processing and the passband of the frequency filter to be applied and to carry out the following operations:
(251) acquiring the signals detected by said at least one sensor (21) and converted in digital ones by said analog/digital conversion means;
(252) obtaining at least a frequency spectrum relative to said signals acquired at point (251);
(253) carrying out notch operations on said signals by eliminating from each signal the frequencies relative to possible interferences deriving from the electric supply;
(254) carrying out frequency filtering operations on said signals, by applying predetermined amplitude band-pass filters;
(255) transmitting the signal obtained through the processing of the previous points to said at least one control module (10).
wherein
the position of the analog/digital conversion means is substantially coincident with the acquisition position, and wherein
between said at least one sensor (21) and said analog/digital conversion means filtration steps or other processing steps are not interposed, except for an amplification step of the signal.

2. System according to claim 1, **characterized in that** said electrode module (20) comprises also temperature sensors (26) and/or humidity sensors (27), configured to detect temperature and humidity relating to the skin region where the electrode is positioned, and **in that** said electronic means (24) are also configured to acquire and process the signals detected by said temperature sensors (26) and/or humidity sensors (27).

3. System according to any one of the preceding claims, **characterized in that** said electrode firmware is also configured to receive in input by said control module (10) and by means of communication means, information about the sampling frequency of the signal by said at least one sensor (21) and the sending frequency of the signal processed to said control module (10).

4. System according to any one of the preceding claims, **characterized in that** said electrode firmware is configured to compare the shape of the frequency spectrum of the signal detected by said at least one sensor (21) with the shape of at least a reference frequency spectrum.

5. System according to any one of the preceding claims, **characterized in that** said firmware is also configured to detect the presence of peaks under 10 Hz of frequency indicating the presence of electrode-skin movements and to send a respective warning signal to said control module (10).

6. System according to any one of the preceding claims, **characterized in that** said firmware is configured to detect periodically a central frequency of said at least one frequency spectrum, and to send a warning signal to said control module in case said central frequency decreases over the time.

7. System according to any one of the preceding claims, **characterized in that** said system comprises a plurality of electrode modules (20), which can be arranged in different positions to each other, with no mutual positioning constraints.

8. System according to any one of the preceding claims, **characterized in that** all the components of said at least one electrode module (20) are enclosed inside an envelope with waterproof and powder resistance features and apt to guarantee its functioning without damage even in presence of natural humidity, splashes or sweating.

9. System according to any one of the preceding claims, **characterized in that** said supplying module (30) comprises at least a rechargeable electric battery, provided with a suitable connector for the recharge by means of connection to the electric network, preferably by means of a common outer voltage transformer with respect to the supplying module.

10. System according to any one of the preceding claims, **characterized in that** said control module (10) comprises:
- control electronic means on which computer programs are loaded;
- digital communication means (11) configured to communicate with other electrodes, and at least an output (13) of analog or digital kind for the control of an actuator;
- communication means, configured to communicate with outer processing means where respective computer programs are loaded configured to allow the user to communicate with said control module (10) by means of a suitable user interface.

11. System according to any one of the preceding claims, **characterized in that** said control module (10) controls directly at least an actuator (40), providing the same with both the control signal and the power needed for supplying moving means included in said actuator, and **in that**
said control module (10) comprises hardware means for electronic control of at least a power actuator and respective computer programs.

12. System according to any one of the preceding claims, **characterized in that** said system comprises also an outer controller configured to receive in input analog and/or digital signals, and to provide the actuator (40) with control signals and the power needed to said moving means,
and **in that**
said control module (10) is configured to control said outer controller.

13. System according to any one of the preceding claims, **characterized in that** said computer programs loaded on said control module (10) are configured for:
- recording the basal signals acquired by the electrode modules (20) during a muscle rest condition of the user,
- adapting the acquisition parameters of the A/D converter on board of the electrode module (20) and the digital filtering parameters, so that the SNR is improved for each electrode module (20) as a function of the recorded basal signals.

14. System according to any one of the preceding claims, **characterized in that** said electromyography sensor (23) is configured to detect a difference of potential between two distinct skin regions.

15. System according to any one of the preceding claims, **characterized in that** said at least one sensor (21) further comprises a force myography sensor, comprising a capacitive pressure sensor configured to measure the pressure exerted on the electrode by the muscle under the skin region where the electrode module is installed.

## Patentansprüche

1. Integriertes System zur Erfassung und Verarbeitung elektromyographischer Signale und zur Steuerung eines Aktuators (40), das mindestens ein mechanisches Bewegungsmittel umfasst und dazu konfiguriert ist, mindestens eine Bewegungsart gemäß mindestens einem Eingangssignal auszuführen,
wobei das System Folgendes umfasst:
- ein Steuermodul (10),
- mindestens ein Elektrodenmodul (20), das zum Erfassen, Verstärken, Umwandeln und Verarbeiten elektromyographischer Signale (20) konfiguriert ist,
- ein Versorgungsmodul (30), das so konfiguriert ist, dass es eine elektrische Versorgung mit einer vorbestimmten Spannung und einem vorbestimmten Strom für das Steuermodul (10) und das mindestens eine Elektrodenmodul (20) bereitstellt,
wobei der Controller (10), das mindestens eine Elektrodenmodul (20) und das Versorgungsmodul (30) parallel an denselben Bus angeschlossen sind,
wobei das Elektrodenmodul (20) Folgendes umfasst:
- mindestens einen Sensor (21), der so konfiguriert ist, dass er ein Signal auf der Hautoberfläche erfasst, das von einer Muskelkontraktion herrührt, wobei der Sensor (21) ein elektromyographischer Sensor (23) mit mindestens zwei auf der Außenseite positionierten Leitungsoberflächen der Hülle des Elektrodenmoduls (20) ist, so dass sie für den Kontakt mit der Haut ausgelegt sind,
- Mittel (22) zum Verstärken des von dem mindestens einen Sensor (21) erfassten Signals,
- Mittel zur Analog/Digital-Umwandlung, die auf einer Leiterplatte installiert sind, die im Inneren der Hülle der Modulelektrode (20) enthalten sind und in elektrischem Kontakt mit dem inneren Teil der Leitungsflächen so konfiguriert ist, dass er das analoge Signal in ein digitales Signal umwandelt, das durch die Verstärkungseinrichtung (22) verstärkt wird,
- elektronische Mittel (24), auf denen Elektroden-Computerprogramme geladen sind, die so konfiguriert sind, dass sie das von dem mindestens einen Sensor (21) erfasste Signal verarbeiten, wobei die Elektroden-Computerprogramme so konfiguriert sind, dass sie im Eingang von den Modulsteuerungen (10) eingegebene Informationen über die Verstärkung und Offset empfangen, die in der Analog/Digital-Umwandlungsphase, bei der Signalverarbeitung zu verwendende Kerbfrequenz und der Band-Pass des anzuwendenden Frequenzfilters verwendet werden sollen, um die folgenden Operationen durchzuführen:
(251) Erfassen der von dem mindestens einen Sensor (21) erfassten und von der Analog/Digital-Umwandlungseinrichtung in digitale Signale umgewandelten Signale;
(252) Erhalten mindestens ein Frequenzspektrum, das sich auf die am Punkt (251) erfassten Signale bezieht;
(253) Durchführen von Kerboperationen an den Signalen durch Eliminieren der Frequenzen aus jedem Signal, die mit möglichen Störungen durch die Stromversorgung verbunden sind;
(254) Durchführen von Frequenzfilterungsschritten an den Signalen durch Anwenden von Bandpassfiltern mit vorgegebener Amplitude;
(255) Übertragen das durch die Verarbeitung der vorstehenden Punkte erhaltene Signal an das mindestens eine Steuermodul (10),
wobei die Position der Analog/Digital-Umwandlungseinrichtung im Wesentlichen mit der Erfassungsposition übereinstimmt und wobei die Position der Analog-/Digital-Umwandlungseinrichtung im Wesentlichen mit der Erfassungsposition übereinstimmt und wobei zwischen dem mindestens einen Sensor (21) und der Analog-/Digital-Umwandlungseinrichtung keine Filterungsphasen oder andere Verarbeitungsphasen, mit Ausnahme einer Verstärkungsphase des Signals, eingefügt sind.

2. System nach Anspruch 1, **dadurch gekennzeichnet, dass** das Elektrodenmodul (20) außerdem Temperatursensoren (26) und/oder Feuchtigkeitssensoren (27) umfasst, die dazu ausgebildet sind, Temperatur und Feuchtigkeit in Bezug auf den Hautbereich zu erfassen, in dem die Elektrode positioniert ist, und dass die elektronischen Mittel (24) außerdem dazu ausgebildet sind, die von den Temperatursensoren (26) und/oder Feuchtigkeitssensoren (27) erfassten Signale zu erfassen und verarbeiten.

3. System nach einem der vorhergehenden Ansprüche, **dadurch gekennzeichnet, dass** die Elektroden-Firmware außerdem so konfiguriert ist, dass sie als Eingabe von dem Steuermodul (10) und mittels der Kommunikationsmittel Informationen über die Abtastfrequenz des Signals durch den mindestens einen Sensor (21) und die Sendefrequenz des an das Steuermodul (10) verarbeiteten Signals empfängt.

4. System nach einem der vorhergehenden Ansprüche, **dadurch gekennzeichnet, dass** die Elektroden-Firmware dazu konfiguriert ist, die Form des Frequenzspektrums des von dem mindestens einen Sensor (21) erfassten Signals mit der Form von mindestens einem Referenzfrequenzspektrum zu vergleichen.

5. System nach einem der vorhergehenden Ansprüche, **dadurch gekennzeichnet, dass** die Firmware außerdem dazu konfiguriert ist, das Vorhandensein von Frequenzspitzen unter 10 Hz zu erkennen, die auf das Vorhandensein von Elektroden-Haut-Bewegungen hinweisen, und ein entsprechendes Warnsignal an das Steuermodul (10) zu senden.

6. System nach einem der vorhergehenden Ansprüche, **dadurch gekennzeichnet, dass** die Firmware so konfiguriert ist, dass sie regelmäßig eine Mittenfrequenz des mindestens einen Frequenzspektrums erkennt und ein Warnsignal an das Steuermodul sendet, wenn die Mittenfrequenz mit der Zeit abnimmt.

7. System nach einem der vorhergehenden Ansprüche, **dadurch gekennzeichnet, dass** das System mehrere Elektrodenmodule (20) umfasst, die ohne gegenseitige Positionierungsbeschränkungen in unterschiedlichen Positionen zueinander angeordnet werden können.

8. System nach einem der vorhergehenden Ansprüche, **dadurch gekennzeichnet, dass** alle Komponenten des mindestens einen Elektrodenmoduls (20) in einer wasserdichten und staubdichten Hülle eingeschlossen sind, die deren Funktion auch bei natürlicher Feuchtigkeit, Spritzern oder Schweiß ohne Beschädigung gewährleistet.

9. System nach einem der vorhergehenden Ansprüche, **dadurch gekennzeichnet, dass** das Versorgungsmodul (30) mindestens eine wiederaufladbare elektrische Batterie umfasst, die mit einem geeigneten Anschluss für das Wiederaufladen mittels Verbindung mit dem Stromnetz, vorzugsweise mittels eines gemeinsamen äußeren Spannungstransformators in Bezug auf das Versorgungsmodul, versehen ist.

10. System nach einem der vorhergehenden Ansprüche, **dadurch gekennzeichnet, dass** das Steuermodul (10) Folgendes umfasst:
- elektronische Steuermittel, auf denen Computerprogramme geladen sind,
- digitale Kommunikationsmittel (11), die zur Kommunikation mit anderen Elektroden konfiguriert sind, und mindestens einen analogen oder digitalen Ausgang (13) zur Steuerung eines Aktuators umfassen;
- Kommunikationsmittel, die zur Kommunikation mit äußeren Verarbeitungsmitteln konfiguriert sind, auf denen entsprechende Computerprogramme geladen sind, die so konfiguriert sind, dass sie dem Benutzer die Kommunikation mit dem Steuermodul (10) mittels einer geeigneten Benutzerschnittstelle ermöglichen.

11. System nach einem der vorhergehenden Ansprüche, **dadurch gekennzeichnet, dass** das Steuermodul (10) mindestens einen Aktuator (40) direkt steuert und diesen sowohl mit dem Steuersignal als auch mit der Energie versorgt, die zur Versorgung der in dem Aktuator enthaltenen Bewegungsmittel erforderlich ist, und dass das Steuermodul (10) Hardwaremittel zur elektronischen Steuerung von mindestens einem Energieaktuator und entsprechende Computerprogramme umfasst.

12. System nach einem der vorhergehenden Ansprüche, **dadurch gekennzeichnet, dass** das System außerdem einen äußeren Controller umfasst, der dazu konfiguriert ist, analoge und/oder digitale Eingangssignale zu empfangen und den Aktuator (40) mit Steuersignalen und der für die Bewegungsmittel erforderlichen Energie zu versorgen, und dass das Steuermodul (10) dazu konfiguriert ist, den äußeren Controller zu steuern.

13. System nach einem der vorhergehenden Ansprüche, **dadurch gekennzeichnet, dass** die auf dem Steuermodul (10) geladenen Computerprogramme dazu konfiguriert sind,
- die von den Elektrodenmodulen (20) während eines Muskelruhezustands des Benutzers erfassten Basalsignale aufzuzeichnen,
- die Erfassungsparameter des A/D-Wandlers an Bord des Elektrodenmoduls (20) und der digitalen Filterparameter anzupassen, so dass das SNR für jedes Elektrodenmodul (20) als Funktion der aufgezeichneten Basalsignale verbessert wird.

14. System nach einem der vorhergehenden Ansprüche, **dadurch gekennzeichnet, dass** der Elektromyographie-Sensor (23) so konfiguriert ist, einen Potentialunterschied zwischen zwei unterschiedlichen Hautbereichen zu erfassen.

15. System nach einem der vorhergehenden Ansprüche, **dadurch gekennzeichnet, dass** der mindestens eine Sensor (21) außerdem einen Kraftmyographie-Sensor umfasst, der einen kapazitiven Drucksensor aufweist, der dazu konfiguriert ist, den Druck zu messen, der vom Muskel unter dem Hautbereich, in dem das Elektrodenmodul installiert ist, auf die Elektrode ausgeübt wird.

## Revendications

1. Système intégré de détection et de traitement de signaux électro-myographiques et de commande d'un actionneur (40), comprenant au moins un moyen de déplacement mécanique et configuré pour effectuer au moins un type de mouvements en fonction d'au moins un signal d'entrée,
ledit système comprenant:
- un module de commande (10),
- au moins un module d'électrode (20) configuré pour acquérir, amplifier, convertir et traiter des signaux électro-myographiques (20),
- un module d'alimentation (30), configuré pour fournir une alimentation électrique avec une tension et un courant prédéterminés audit module de commande (10) et audit au moins un module d'électrode (20),
ledit contrôleur (10), ledit au moins un module d'électrode (20) et ledit module d'alimentation (30) étant connectés en parallèle sur le même bus,
dans lequel ledit module d'électrode (20) comprend:
- au moins un capteur (21) configuré pour détecter un signal sur la surface de la peau provenant de la contraction musculaire, ledit capteur (21) étant un capteur électro-myographique (23), avec au moins deux surfaces de conduction positionnées sur le côté externe de l'enveloppe dudit module d'électrode (20), de telle sorte qu'elles sont configurées pour se trouver en contact avec la peau,
- des moyens d'amplification (22) du signal détecté par ledit au moins un capteur (21),
- des moyens de conversion analogique/numérique, installés sur une base de circuit imprimé contenue à l'intérieur de l'enveloppe dudit module d'électrode (20) et en contact électrique avec la partie interne des dites surfaces de conduction configurées pour convertir le signal analogique en signal numérique, amplifié par lesdits moyens d'amplification (22),
- des moyens électroniques (24) sur lesquels sont chargés des programmes informatiques d'électrodes, configurés pour traiter le signal acquis par ledit au moins un capteur (21), ledit programme informatique d'électrodes étant configuré pour recevoir en entrée par ledit module de commande (10) des informations sur le gain et le décalage à utiliser dans la phase de conversion analogique/numérique, la fréquence d'encoche à utiliser dans le traitement du signal, et la passe-bande du filtre de fréquence à appliquer, pour effectuer les opération suivantes:
(251) acquérir les signaux détectés par ledit au moins un capteur (21) et convertis en signaux numériques par lesdits moyens de conversion analogique/numérique;
(252) obtenir au moins un spectre de fréquences relatif auxdits signaux acquis au point (251);
(253) effectuer des opérations d'encoche sur lesdits signaux en éliminant de chaque signal les fréquences relatives à des possibles interférences dérivées de l'alimentation électrique;
(254) effectuer des opérations de filtrage fréquentiel sur lesdits signaux, en appliquant des filtres passe-bande d'amplitude prédéterminée;
(255) transmettre le signal obtenu grâce au traitement des points précédents audit au moins un module de commande (10), dans lequel
la position des moyens de conversion analogique/numérique coïncide sensiblement avec la position d'acquisition et dans lequel entre ledit au moins un capteur (21) et lesdites phases de filtrage, des moyens de conversion analogique/numérique ou d'autres phases de traitement ne sont pas interposés, sauf pour une phase d'amplification du signal.

2. Système selon la revendication 1, **caractérisé en ce que** ledit module d'électrode (20) comprend également des capteurs de température (26) et/ou des capteurs d'humidité (27), configurés pour détecter la température et l'humidité relatives à la région de la peau où est positionnée l'électrode, et **en ce que** lesdits moyens électroniques (24) sont également configurés pour acquérir et traiter les signaux détectés par lesdits capteurs de température (26) et/ou par des capteurs d'humidité (27).

3. Système selon l'une quelconque des revendications précédentes, **caractérisé en ce que** ledit programme informatique d'électrode est également configuré pour recevoir en entrée par ledit module de commande (10) et par lesdits moyens de communication, des informations sur la fréquence d'échantillonnage du signal par ledit au moins un capteur (21) et la fréquence d'envoi du signal traité audit module de commande (10).

4. Système selon l'une quelconque des revendications précédentes, **caractérisé en ce que** ledit programme informatique d'électrode est configuré pour comparer la forme du spectre de fréquences du signal détecté par ledit au moins un capteur (21) avec la forme d'au moins un spectre de fréquences de référence.

5. Système selon l'une quelconque des revendications précédentes, **caractérisé en ce que** ledit programme informatique est également configuré pour détecter la présence de pics inférieurs à 10 Hz de fréquence indiquant la présence de mouvements électrode - peau et pour envoyer un signal d'avertissement respectif audit module de commande (10).

6. Système selon l'une quelconque des revendications précédentes, **caractérisé en ce que** ledit programme informatique est configuré pour détecter périodiquement une fréquence centrale dudit au moins un spectre de fréquences, et pour envoyer un signal d'avertissement audit module de commande au cas où ladite fréquence centrale diminue avec le temps.

7. Système selon l'une quelconque des revendications précédentes, **caractérisé en ce que** ledit système comprend une pluralité de modules d'électrodes (20), qui peuvent être disposés dans des positions différentes les unes par rapport aux autres, sans contraintes de positionnement mutuel.

8. Système selon l'une quelconque des revendications précédentes, **caractérisé en ce que** tous les composants dudit au moins un module d'électrode (20) sont enfermés à l'intérieur d'une enveloppe présentant des caractéristiques d'étanchéité et de résistance à l'eau et à la poudre et apte à garantir son fonctionnement sans dommage même en présence d'humidité naturelle, des éclaboussures ou de la transpiration.

9. Système selon l'une quelconque des revendications précédentes, **caractérisé en ce que** ledit module d'alimentation (30) comprend au moins une batterie électrique rechargeable, munie d'un connecteur approprié pour la recharge au moyen de connexion au réseau électrique, de préférence au moyen d'un commun transformateur de tension externe par rapport au module d'alimentation.

10. Système selon l'une quelconque des revendications précédentes, **caractérisé en ce que** ledit module de commande (10) comprend:
- des moyens électroniques de commande sur lesquels sont chargés des programmes informatiques;
- des moyens de communication numérique (11) configurés pour communiquer avec d'autres électrodes, et au moins une sortie (13) de type analogique ou numérique pour la commande d'un actionneur;
- des moyens de communication, configurés pour communiquer avec des moyens de traitement externes dans lesquels des programmes informatiques respectifs sont chargés, configurés pour permettre à l'utilisateur de communiquer avec lesdits modules de commande (10) au moyen d'une interface utilisateur appropriée.

11. Système selon l'une quelconque des revendications précédentes, **caractérisé en ce que** ledit module de commande (10) commande directement au moins un actionneur (40), lui fournissant à la fois le signal de commande et la puissance nécessaire à l'alimentation des moyens mobiles inclus dans ledit actionneur, et **en ce que** ledit module de commande (10) comprend des moyens matériels pour commander électroniquement au moins un actionneur de puissance et des programmes informatiques respectifs.

12. Système selon l'une quelconque des revendications précédentes, **caractérisé en ce que** ledit système comprend également un contrôleur externe configuré pour recevoir en entrée des signaux analogiques et/ou numériques, et pour fournir à l'actionneur (40) des signaux de commande et la puissance nécessaire audit des moyens de mouvement, et **en ce que** ledit module de commande (10) est configuré pour commander ledit contrôleur externe.

13. Système selon l'une quelconque des revendications précédentes, **caractérisé en ce que** lesdits programmes informatiques chargés sur ledit module de commande (10) sont configurés pour:
- enregistrer les signaux basaux acquis par les modules d'électrodes (20) lors d'une condition de repos musculaire de l'utilisateur,
- adapter les paramètres d'acquisition du convertisseur A/D embarqué sur le module d'électrodes (20) et les paramètres de filtrage numérique, de sorte que le SNR soit amélioré pour chaque module d'électrodes (20) en fonction des signaux basaux enregistrés.

14. Système selon l'une quelconque des revendications précédentes, **caractérisé en ce que** ledit au moins un capteur électro-myographique (23) est configuré pour détecter une différence de potentiel entre deux régions distinctes de la peau.

15. Système selon l'une quelconque des revendications précédentes, **caractérisé en ce que** ledit au moins un capteur (21) comprend en outre un capteur de force myographique, comprenant un capteur de pression capacitif configuré pour mesurer la pression exercée sur l'électrode par le muscle sous la région de la peau, où le module d'électrodes est installé.
